(19) Europäisches Patentamt
European Patent Office
Office européen des brevets

(11) Publication number: **0 187 012**
**B1**

(12) **EUROPEAN PATENT SPECIFICATION**

(45) Date of publication of patent specification: **21.06.89**

(51) Int. Cl.$^4$: **A 61 K 31/55** // C07D487/04, C07D498/04

(21) Application number: **85309163.5**

(22) Date of filing: **16.12.85**

(54) **Novel compositions.**

(30) Priority: **22.12.84 GB 8432576**
**21.02.85 GB 8504463**

(43) Date of publication of application:
**09.07.86 Bulletin 86/28**

(45) Publication of the grant of the patent:
**21.06.89 Bulletin 89/25**

(84) Designated Contracting States:
**BE CH DE FR GB IT LI NL**

(56) References cited:
**EP-A-0 126 343**
**EP-A-0 134 984**
**FR-A-2 461 495**
**GB-A-1 128 275**
**US-A-4 447 414**

(73) Proprietor: **BEECHAM GROUP PLC**
**Beecham House Great West Road**
**Brentford Middlesex TW8 9BD (GB)**

(72) Inventor: **Brewer, Malcolm David**
**Stuart House High Street**
**Handcross West Sussex (GB)**
Inventor: **Dorgan, Roderick John**
**Lingworth Scotts Hill**
**Outwood Surrey (GB)**
Inventor: **Elliott, Richard Leonard**
**18 Little Bookham Street Great Bookham**
**Leatherhead Surrey (GB)**
Inventor: **Mamalis, Patrick**
**"Robins Ruff" 7 Wraylands Drive**
**Reigate Surrey (GB)**
Inventor: **Manger, Brian Richard**
**24 Braes Mead South Nutfield**
**Surrey, RH1 4JR (GB)**

(74) Representative: **Lockwood, Barbara Ann et al**
**Beecham Pharmaceuticals Biosciences Research Centre Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 187 012 B1**

**Description**

The present invention relates to pharmaceutical compositions, in particular to compositions having broad spectrum anthelmintic activity, and their use for the manufacture of a medicament for the treatment of helminthiasis in human beings and domestic animals.

Accordingly, the present invention provides a pharmaceutical composition comprising a compound of formula (I):

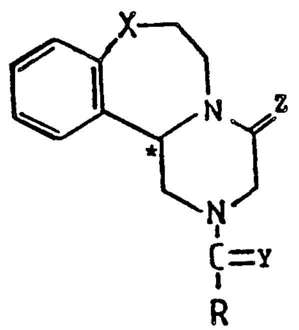

(I)

in which R is optionally substituted phenyl, $C_{3-8}$ cycloalkyl, $C_{5-8}$ cycloalkenyl, $C_{1-8}$ alkyl which may be straight or branched, $C_{2-8}$ alkenyl which may be straight or branched, 5- or 6-membered heterocyclyl, or optionally substituted phenyl $C_{1-4}$ alkyl; Y and Z, which may be the same or different, represent oxygen or sulphur; and X is $-CH_2-$ or oxygen; and at least one other anthelmintically active compound, selected from fenbendazole, avermectin, piperazine, morantel, pyrantel, levamisole and pharmaceutically acceptable salts thereof, optionally together with a pharmaceutically acceptable carrier.

Compounds of formula (I) are disclosed in the earlier European Patent Application 0 134 984.

Suitably, the said other anthelmintically active compound has anthelmintic activity against nematodes, especially against hook worms and ascarid worms in man or domestic animals, for example dogs and cats.

Suitable compounds with anthelmintic activity are fenbendazole, avermectin, piperazine, morantel, pyrantel or levamisole.

Some of the anthelmintically active compounds form salts; it will be understood therefore that the composition of the present invention may include a pharmaceutically acceptable salt of these compounds.

Suitable salts are the acid addition salts, for example the pharmaceutically acceptable salts of acids such as hydrochloric, hydrobromic, hydroiodic, nitric, sulphuric, citric, tartaric and pamoic acids.

A preferred compound is pyrantel or a pharmaceutically acceptable salt thereof, in particular pyrantel pamoate.

When used herein the term "pharmaceutical" includes the term "veterinary" and the term "pharmaceutically" includes the term "veterinarily".

Compounds of formula (I) have an asymmetric carbon atom (marked by an asterisk) and may therefore exist in at least two stereoisomeric forms. The present invention encompasses all isomers of the compounds of formula (I) whether pure or admixed with other isomers in any proportion.

When R is optionally substituted phenyl, the phenyl group may be substituted with up to three groups selected from halogen, $C_{1-6}$ alkyl, $C_{1-6}$ alkoxy, nitro, amino, mono- or di-$C_{1-6}$ alkylamino, and hydroxy.

When R is heterocyclyl, it may be a 5 or 6-membered saturated or unsaturated group containing up to three hetero-atoms selected from oxygen, sulphur and nitrogen. It will be appreciated that unsaturated heterocyclyl groups suitably include aromatic heterocyclyl groups.

The term "halogen" refers to fluorine, chlorine, bromine and iodine.

A preferred R group is cyclohexyl.

Compounds of formula (I) may be produced by cyclising a compound of formula (II):

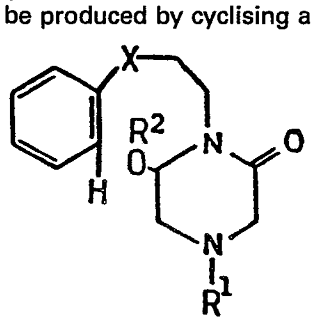

(II)

in which

X is as defined in formula (I);

$R^1$ is hydrogen, a protecting group or a group

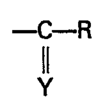

wherein R and Y are as defined in formula (I); and

$R^2$ is $C_{1-3}$ alkyl or hydrogen;

and thereafter, if necessary:

i) removing any protecting group $R^1$ and replacing it with a group

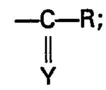

or

ii) when $R^1$ is hydrogen, replacing it with a group

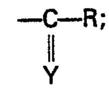

and/or

iii) converting the compound of formula (I) thus formed, wherein Z is oxygen, to a compound of formula (I) wherein Z is sulphur, by treatment with a thionation reagent.

As used herein the term "thionation reagent" denotes a reagent capable of converting the group

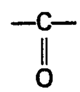

into the group

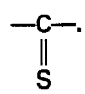

A preferred thionation reagent is Lawesson's reagent, *i.e.* 2,4-bis(4-methoxyphenyl)-1,3-dithia-2,4-diphosphetane-2,4-disulphide, as described, for example, in Synthesis, 941 (1979), Tetrahedron *35,* 2433 (1979) and Tetrahedron Letters, *21,* 4061 (1980).

In the above process, replacement of the group $R^1$ [after the compound of formula (II) has been cyclised] by the group

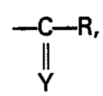

in which Y is sulphur, may be carried out using a dithioic ester of formula

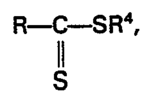

wherein R is hereinbefore defined and $R^4$ is $C_{1-6}$ alkyl.

As used herein the term 'protecting group' refers to a group which is stable under the cyclisation reaction conditions but which may readily be removed after the cyclisation is complete. A typical example of such a protecting group is benzyl, which may be removed by catalytic hydrogenation, for instance using a palladium catalyst in a suitable solvent, for example ethanol.

Examples of $R^2$ are methyl, ethyl, propyl and hydrogen; preferably $R^2$ is hydrogen.

Compounds of formula (II) may be cyclised by treatment with an acid catalyst, and conveniently an acid such as polyphosphoric acid may be used. The reaction may be conducted at elevated temperature, such as 100°C or greater, for instance at about 180°C. Alternatively, concentrated sulphuric acid may be used, in which case the reaction is carried out at a lower temperature, for example from −10°C to +40°C.

Compounds of formula (II) wherein $R^2$ is hydrogen may be produced by reducing the corresponding imide of formula (III):

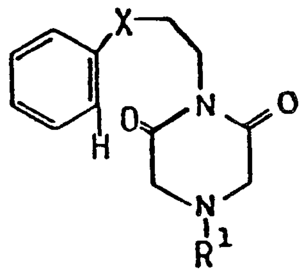

(III)

wherein X, and $R^1$ are as hereinbefore defined. The reduction is effected using a suitable hydride reducing agent, such as sodium borohydride in a suitable solvent such as a lower alkanol, preferably ethanol.

Compounds of formula (II) wherein $R^2$ is alkyl may be produced by conventional methods, such as those outlined in the papers of W. Speckamp *et al* [for example, see Tetrahedron *31*, 1437 (1975)].

Compounds of formula (III) may be produced according to Scheme I, using conventional reagents, such as those shown in the scheme.

Scheme I

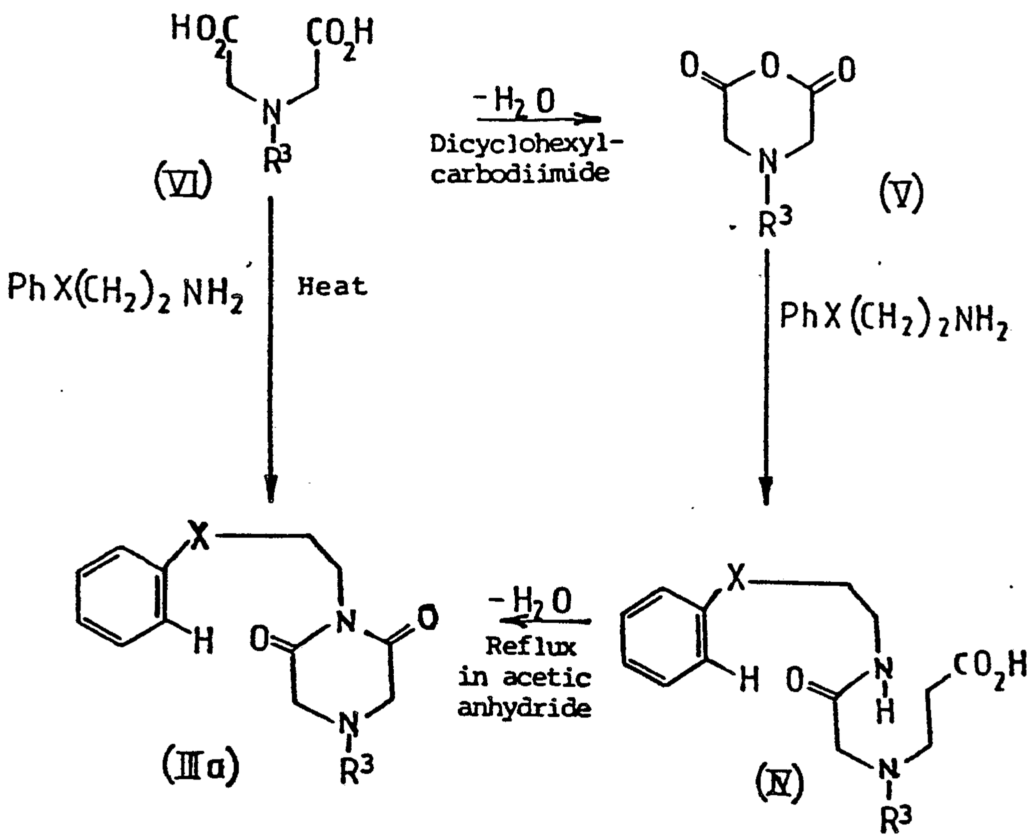


In Scheme I, $R^3$ is a protecting group or a group

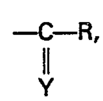

in which Y is as hereinbefore defined, and X is as hereinbefore defined.

It will be appreciated that the compounds of formula (IIIa) in Scheme I have the same structure as compounds of formula (III) as hereinbefore defined except when $R^1$ in the latter is hydrogen.

Compounds of formula (III) wherein $R^1$ is hydrogen may be produced by removing a protecting group $R^3$ from a compound of formula (IIIa).

Compounds of formula (I) have anthelmintic activity especially against tapeworm such as *Taenia taeniaeformis* and *Dipylidium caninum*.

The pharmaceutical composition of the invention is of use in the treatment of helminthiasis of the human or non-human animal body, and particularly for treating tapeworm and/or nematode infestations of domestic animals, especially dogs and cats and farm animals.

Suitably, the pharmaceutical composition comprises a pharmaceutically acceptable carrier or vehicle, the particular carrier used depending upon the chosen means of administration.

Suitable carriers are those used conventionally in the art for the particular means of administration. Thus, for example, if the pharmaceutical composition is to be formulated as a bolus, tablet or capsule, the pharmaceutically acceptable carrier will be chosen from the usual range of lubricants, dispersants, binders and fillers.

For administration to humans, especially children, the pharmaceutical composition may suitably be formulated as a syrup including suitable colouring and/or flavouring agents. Such syrups are conveniently presented in unit or multi-dose containers.

For veterinary use the pharmaceutical composition may be formulated as a dispersion or a solution of the composition in a suitable vehicle for use with an oral doser (a well known item of veterinary or farm equipment, comprising a liquid reservoir, a mouthpiece adapated for insertion into animals' mouths, and a pump mechanism whereby unit doses can be ejected from the reservoir through the mouthpiece). Conveniently the pharmaceutical composition may be administered from the oral doser in the form of an aqueous suspension. Alternatively, the carrier included in the composition of the invention will be an oil or water based cream to ensure homogeneity of the unit doses administered.

The pharmaceutical composition may also be added to the animal feed or drinking water so that the animal ingests an appropriate quantity of the pharmaceutical composition with its diet. In this case the composition is conveniently presented as a premix containing several doses.

The pharmaceutical composition may also be formulated for injection. In such cases the pharmaceutical composition chosen is suitably dissolved or suspended in a suitable pharmaceutically acceptable vehicle, for example water, propylene glycol or glycerol formal.

Suitably the pharmaceutical composition comprises sufficient material to provide a dose of from 0.01 to 250 mg of the compound of formula (I) per kg of animal body weight per dose, more suitably 0.1 to 50 mg/kg per dose. The said other anthelmintically active compounds are present in the pharmaceutical composition in an amount sufficient to provide a dose within a range conventional for the particular compound.

The following examples illustrate the invention.

### Preparation 1

1-(3-Phenylpropyl)-4-benzyl-2,6-piperazinedione

3-Phenyl-1-propylamine (3.63 g) and N-benzyliminodiacetic acid (6.0 g) was mixed and heated to 200°C under a nitrogen atmosphere. The mixture was stirred at this temperature for 1 h, cooled and purified by column chromatography ($SiO_2$, 40—60°C petroleum ether/chloroform) to give the title material as a pale orange liquid (5.53 g, 64%).

### Preparation 2

1-(3-Phenylpropyl)-4-benzyl-2-hydroxy-6-oxopiperazine

Saturated aqueous sodium bicarbonate solution (20 ml) was added to a solution of 1-(3-phenylpropyl)-4-benzyl-2,6-piperazinedione (5.35 g) in ethanol (170 ml) at 5°C and sodium borohydride (1.23 g) added portionwise to the resulting mixture at 5°C over a period of 2 h. The mixture was stirred for a further 1 h at 5°C and the solvent removed *in vacuo*. Water (50 ml) was added, and the mixture extracted with dichloromethane (3 × 50 ml), the extracts washed with brine and dried ($MgSO_4$). Evaporation of the solvent gave the title compound as a white solid (4.36 g, 81%).

### Preparation 3

2-Benzyl-4-oxo-1,2,3,4,6,7,8,12b-octahydropyrazino[2,1-*a*][2]benzazepine

1-(3-Phenylpropyl)-4-benzyl-2-hydroxy-6-oxopiperazine (2.6 g) and polyphosphoric acid (53 g) were mixed, heated at 180°C and maintained at this temperature for ¾ h. The mixture was cooled to 60°C and water (200 ml) added. The mixture was cooled, basified with sodium hydroxide solution, and extracted with chloroform (3 × 50 ml). The solvent was evaporated and the product crystallised from diethyl ether to give the title compound (0.78 g, 32%) m.p. 125—8°C.

### Preparation 4

4-Oxo-1,2,3,4,6,7,8,12b-octahydropyrazino[2,1-*a*][2]benzazepine

Hydrogenolysis of 2-benzyl-4-oxo-1,2,3,4,6,7,8,12b-octahydropyrazino[2,1-*a*][2]benzazepine (1.39 g) in solution in ethanol (40 ml) by treatment with hydrogen at 45°C and atmospheric pressure in the presence of a palladium on charcoal catalyst (0.3 g) gave the title compound (0.6 g, 61%).

Preparation 5

Resolution of 2H-4-oxo-1,2,3,4,6,7,8,12b-octahydropyrazino[2,1-*a*][2]benzazepine

(±) 2H-4-oxo-1,2,3,4,6,7,8,12b-octahydropyrazino[2,1-*a*][2]benzazepine (3.2 g, 0.0148 mol) was dissolved in methanol (35 ml) and a solution of (−) tartaric acid (2.45 g, 0.0163 mol) in methanol (140 ml) added.

The mixture was heated on a steam bath, filtered whilst hot, and allowed to cool. White crystals were deposited, and these were filtered, and recrystallised from methanol (250 ml) to give the (−) tartarate salt of (−) 2H-4-oxo-1,2,3,4,6,7,8,12b-octahydropyrazino[2,1-*a*][2]benzazepine $(\alpha)_D^{22}$ −149° ($H_2O$).

A solution of this salt in water gave, upon basification with ammonium hydroxide and extraction with chloroform, the free base (−) 2H-4-oxo-1,2,3,4,6,7,8,12b-octahydropyrazino[2,1-*a*][2]benzazepine as a white solid $(\alpha)_D^{22}$ −221° ($CH_3OH$).

Similarly, using (+) tartaric acid in place of (−) tartaric acid was obtained:—

(+) 2H-4-oxo-1,2,3,4,6,7,8,12b-octahydropyrazino[2,1-*a*][2]benzazepine (+) tartrate, $(\alpha)_D^{22}$ +153° ($H_2O$) (Found C; 55.7, H; 6.1, N; 7.4%. $C_{17}H_{22}N_2O_7$ requires C; 55.7, H; 6.0, N; 7.6%), and

(+) 2H-4-oxo-1,2,3,4,6,7,8,12b-octahydropyrazino[2,1-*a*][2]benzazepine free base $(\alpha)_D^{22}$ +212° ($CH_3OH$).

Preparation 6

2-(Cyclohexylcarbonyl)-4-oxo-1,2,3,4,6,7,8,12b-octahydropyrazino[2,1-*a*][2]benzazepine

Cyclohexanoyl chloride (0.34 g) was added to a solution of 4-oxo-1,2,3,4,6,7,8,12*b*-octahydropyrazino-[2,1-*a*][2]benzazepine (0.5 g) in chloroform (20 ml ethanol free) maintained at 0°C and triethylamine (0.26 g) added. The mixture was maintained at 0°C for 30 min then at room temperature for 5 h. The solution was washed with, first, dilute hydrochloric acid and secondly with sodium bicarbonate solution. The chloroform solution was dried ($MgSO_4$) and evaporated. The residue was recrystallised from chloroform/40—60°C petroleum ether to give white crystals of the title compound (0.48 g, 64%) m.p. 187—90°C.

| | | | |
|---|---|---|---|
| Found: | C: 73.2, | H: 7.7, | N: 8.4% |
| $C_{20}H_{26}N_2O_2$ requires | C: 73.6, | H: 8.0, | N: 8.6% |

Preparation 7

Pyrantel pamoate

Pyrantel pamoate may be prepared as described in Belgian Patent No. 658987.

Preparations 8—10

The following tabulated preparations were carried out in analogous fashion to that described in Preparation 6.

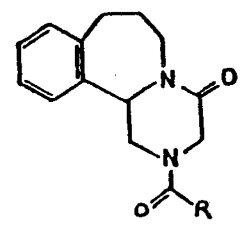

| Preparation No. | R | m.p. °C | Microanalytical Data | | | | | | | |
|---|---|---|---|---|---|---|---|---|---|---|
| | | | Calculated | | | | Found | | | |
| | | | C | H | N | | C | H | N | |
| 8 | 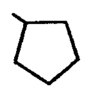 | 154-155 | 73.05 | 7.74 | 8.97 | | 72.31 | 7.43 | 8.88 | |
| 9 | 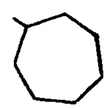 | 160-165 | 74.08 | 8.29 | 8.23 | | 74.27 | 8.30 | 8.14 | |
| 10 | 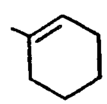 | 149-150 | 74.04 | 7.46 | 8.63 | | 73.97 | 7.46 | 8.61 | |

Preparation 11

2-(Cyclohexylcarbonyl)-4-oxo-1,2,3,6,7,12*b*-hexahydropyrazino[1,2-d][1,4]benzoxazepine

4-Oxo-1,2,3,6,7,12*b*-hexahydropyrazino[1,2-d][1,4]benzoxazepine (0.9 g) and triethylamine (1 g) were dissolved in dichloromethane (20 ml), cooled in ice and cyclohexanoyl chloride (0.7 g, 1.1 equiv.) added. The mixture was stirred at room temperature for 3 h, then washed with dilute aqueous hydrochloric acid, followed by dilute aqueous ammonia solution. Evaporation of the dichloromethane gave an oil which was purified by column chromatography ($SiO_2$/chloroform) and crystallised from ether/40°—60° petroleum ether to give white crystals of the title compound m.p. 92—3°;

| | | | |
|---|---|---|---|
| found: | C: 69.53 | H: 7.40, | N: 8.54% |
| $C_{19}H_{24}N_2O_3$ requires | C: 69.49 | H: 7.37, | N: 8.53% |

Preparation 12

(+) 2-(Cyclohexylcarbonyl)-4-oxo-1,2,3,4,6,7,8,12b-octahydropyrazino[2,1-*a*][2]benzazepine

The title compound was obtained by the method of preparation 1 using (+) 2H-4-oxo-1,2,3,4,6,7,8,12b-octahydropyrazino[2,1-*a*][2]benzazepine in place of the racemic compound. The product was purified by column chromatography ($SiO_2/CHCl_3$). $(\alpha)_D^{22}$ +41° ($CH_3OH$).

Preparation 13

(−) 2-(Cyclohexylcarbonyl)-4-oxo-1,2,3,4,6,7,8,12b-octahydropyrazino[2,1-*a*][2]benzazepine

The title compound was obtained as a white solid by the procedure outlined in preparation 1 using (−) 2H-4-oxo-1,2,3,4,6,7,8,12b-octahydropyrazino[2,1-*a*][2]benzazepine in place of the racemic compound. The product was purified by column chromatography ($SiO_2/CHCl_3$). $(\alpha)_D^{22}$ −42° ($CH_3OH$).

The following are examples of pharmaceutical compositions of the invention.

Example 1

| | % (w/w) |
|---|---|
| 2-(Cyclohexylcarbonyl)-4-oxo-1,2,3,4,6,7,8,12b-octahydropyrazino[2,1-*a*][2]benzazepine | 25 |
| Pyrantel pamoate | 75 |

Example 2

| | % (w/w) |
|---|---|
| 2-(Cyclopentylcarbonyl)-4-oxo-1,2,3,4,6,7,8,12b-octahydropyrazino[2,1-*a*][2]benzazepine | 25 |
| Pyrantel pamoate | 75 |

Example 3

| | % (w/w) |
|---|---|
| 2-(Cycloheptylcarbonyl)-4-oxo-1,2,3,4,6,7,8,12b-octahydropyrazino[2,1-*a*][2]benzazepine | 25 |
| Pyrantel pamoate | 75 |

Example 4

| | % (w/w) |
|---|---|
| 2-(Cyclohexenylcarbonyl)-4-oxo-1,2,3,4,6,7,8,12b-octahydropyrazino[2,1-*a*][2]benzazepine | 25 |
| Pyrantel pamoate | 75 |

Example 5

| | % (w/w) |
|---|---|
| 2-(Cyclohexylcarbonyl)-4-oxo-1,2,3,6,7,12b-hexahydropyrazino[1,2-*a*][2]benzoxazepine | 25 |
| Pyrantel pamoate | 75 |

Example 6

| | % (w/w) |
|---|---|
| (−) 2-(Cyclohexylcarbonyl)-4-oxo-1,2,3,4,6,7,8,12b-octahydropyrazino[2,1-*a*][2]benzazepine | 25 |
| Pyrantel pamoate | 75 |

Pharmacological Data

a) A cat infected with *Toxascaris leonina* and *Dipylidium caninum* was orally dosed with sufficient of the composition of Example 1 (formulated as gelatin capsule) to give a dose of 5 mg/kg of 2-(cyclohexylcarbonyl)-4-oxo-1,2,3,4,6,7,8,12b-octahydropyrazino[2,1-a]benzazepine and 15 mg/kg of pyrantel pamoate. This treatment completely cleared the animal of both infections.

b) Three cats infected with *Toxocara cati* in addition to an infection of *Dipylidium caninum, Taenia taeniaeformis* and a combination of the two respectively were dosed orally with gelatine capsules containing a mixture of 2-(cyclohexylcarbonyl)-4-oxo-1,2,3,4,6,7,8,12*b*-octahydropyrazino[2,1-*a*]benzazepine and pyrantel pamoate to give a dose of 7.5 mg/kg of the former and 55 mg/kg of the latter. All three cats were completely cleared of their parasitic infections.

**Claims**

1. A pharmaceutical composition comprising a compound of formula (I):

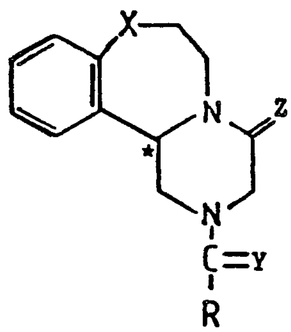

(I)

in which R is phenyl optionally substituted with up to three groups selected from halogen, $C_{1-6}$ alkyl, $C_{1-6}$ alkoxy, nitro, amino, mono- or di-$C_{1-6}$ alkylamino and hydroxy, $C_{3-8}$ cycloalkyl, $C_{5-8}$ cycloalkenyl, $C_{1-8}$ alkyl which may be straight or branched, $C_{2-8}$ alkenyl which may be straight or branched, 5- or 6-membered heterocyclyl, or phenyl $C_{1-4}$ alkyl in which the phenyl group is optionally substituted with up to three groups selected from halogen, $C_{1-6}$ alkyl, $C_{1-6}$ alkoxy, nitro, amino, mono- or di-$C_{1-6}$ alkylamino and hydroxy; Y and Z, which may be the same or different, represent oxygen or sulphur; and X is $—CH_2—$ or oxygen; and at least one other anthelmintically active compound selected from the group consisting of fenbendazole, avermectin, piperazine, morantel, pyrantel, levamisole and pharmaceutically acceptable salts thereof, optionally together with a pharmaceutically acceptable carrier.

2. A pharmaceutical composition as claimed in claim 1 in which in the compound of formula (I) R is $C_{3-8}$ cycloalkyl or $C_{5-8}$ cycloalkenyl.

3. A pharmaceutical composition as claimed in claim 1 or claim 2 in which in the compound of formula (I) Y and Z are both oxygen.

4. A pharmaceutical composition as claimed in any one of claims 1 to 3 in which the other anthelmintically active compound is pyrantel or a pharmaceutically acceptable salt thereof.

5. A pharmaceutical composition as claimed in claim 4 in which the other anthelmintically active compound is pyrantel pamoate.

6. A pharmaceutical composition comprising 2-(cyclohexylcarbonyl)-4-oxo-1,2,3,4,6,7,8,12b-octahydropyrazino[2,1-*a*][2]benzazepine and pyrantel or a pharmaceutically acceptable salt thereof, together with a pharmaceutically acceptable carrier.

7. A pharmaceutical composition as claimed in claim 6 in which pyrantel is in the form of the pamoate salt.

8. A pharmaceutical composition as claimed in any one of claims 1 to 7, adapted for oral administration.

9. A pharmaceutical composition as claimed in claim 8 in the form of a bolus, tablet or capsule.

10. The use of a compound of formula (I) as claimed in claim 1, and at least one other anthelmintically active compound selected from the group consisting of fenbendazole, avermectin, piperazine, morantel, pyrantel, levamisole and pharmaceutically acceptable salts thereof, for the manufacture of a medicament for the treatment of helminthiasis in human beings and domestic animals.

## Patentansprüche

1. Eine pharmazeutische Zusammensetzung umfassend eine Verbindung der Formel (I):

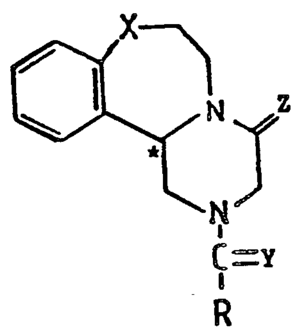

(I)

in welcher R Phenyl, gegebenenfalls substituiert mit bis zu drei Gruppen, ausgewählt aus Halogen, $C_{1-6}$-Alkyl, $C_{1-6}$-Alkoxy, Nitro, Amino, Mono- oder Di-$C_{1-6}$-alkylamino und Hydroxy; $C_{3-8}$-Cycloalkyl, $C_{5-8}$-Cycloalkenyl, $C_{1-8}$-Alkyl, welches geradkettig oder verzweigt sein kann, $C_{2-8}$-Alkenyl, welches geradkettig oder verzweigt sein kann, 5- oder 6-gliedriges Heterocyclyl oder Phenyl-$C_{1-4}$-alkyl, wobei die Phenylgruppe gegebenenfalls substituiert ist mit bis zu drei Gruppen, ausgewählt aus Halogen, $C_{1-6}$-Alkyl, $C_{1-6}$-Alkoxy, Nitro, Amino, Mono- oder Di-$C_{1-6}$-alkylamino und Hydroxy, darstellt; Y und Z, welche gleich oder verschieden sein können, Sauerstoff oder Schwefel darstellen; und X —$CH_2$— oder Sauerstoff bedeutet; und mindestens eine weitere anthelmintisch wirkende Verbindung, ausgewählt aus der Gruppe bestehend aus Fenbendazol, Avermectin, Piperazin, Morantel, Pyrantel, Levamisol und deren pharmazeutisch verträglichen Salzen, gegebenenfalls zusammen mit einem pharmazeutisch verträglichen Träger.

2. Eine pharmazeutische Zusammensetzung wie in Anspruch 1 beansprucht, in welcher in der Verbindung der Formel (I) R $C_{3-8}$-Cycloalkyl oder $C_{5-8}$-Cycloalkenyl ist.

3. Eine pharmazeutische Zusammensetzung wie in Anspruch 1 oder 2 beansprucht, in welcher in der Verbindung der Formel (I) Y und Z jeweils Sauerstoff bedeuten.

4. Eine pharmazeutische Zusammensetzung wie in einem der Ansprüche 1 bis 3 beansprucht, in welcher die andere anthelmintisch wirkende Verbindung Pyrantel oder ein pharmazeutisch verträgliches Salz davon ist.

5. Eine pharmazeutische Zusammensetzung wie in Anspruch 4 beansprucht, in welcher die andere anthelmintisch wirkende Verbindung Pyrantelpamoat (Salz der Embonsäure) ist.

6. Eine pharmazeutische Zusammensetzung umfassend 2-(Cyclohexyl carbonyl)-4-oxo-1,2,3,4,-6,7,8,12b-octahydropyrazino[2,1-a][2]benzazepin und Pyrantel oder ein pharmazeutisch verträgliches Salz davon, zusammen mit einem pharmazeutisch verträglichen Träger.

7. Eine pharmazeutische Zusammensetzung wie in Anspruch 6 beansprucht, in welcher Pyrantel in der Form des Embonsäuresalzes vorliegt.

8. Eine pharmazeutische Zusammensetzung wie in einem der Ansprüche 1 bis 7 beansprucht, die für die orale Verabreichung geeignet ist.

9. Eine pharmazeutische Zusammensetzung wie in Anspruch 8 beansprucht, welche in Bolus-, Tabletten- oder Kapselform vorliegt.

10. Die Verwendung einer Verbindung der Formel (I) wie in Anspruch 1 beansprucht, und mindestens einer weiteren anthelmintisch wirkenden Verbindung, ausgewählt aus der Gruppe bestehend aus Fenbendazol, Avermectin, Piperazin, Morantel, Pyrantel, Levamisol und pharmazeutisch verträglichen Salzen davon für die Herstellung eines Medikaments zur Behandlung von Helminthiasis bei Mensch und Tier.

## Revendications

1. Composition pharmaceutique, caractérisée en ce qu'elle comprend un composé de formule (I):

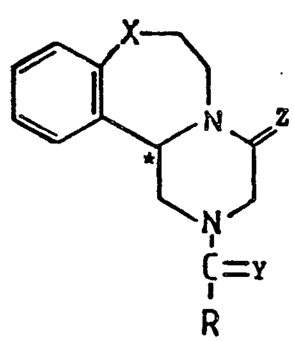

(I)

dans laquelle R est un groupe phényle éventuellement substitué avec jusqu'à trois groupes choisis parmi un atome d'halogène, un groupe alkyle en $C_{1-6}$, alkoxy en $C_{1-6}$, nitro, amino, mono- ou di-alkyle($C_{1-6}$)amino et hydroxy, cycloalkyle en $C_{3-8}$, cycloalcényle en $C_{5-8}$, alkyle en $C_{1-8}$ à chaîne droite ou ramifiée, alcényle en $C_{2-8}$ à chaîne droite ou ramifiée, hétérocyclyle à 5 ou 6 chaînons ou phénylalkyle en $C_{1-4}$, dans lequel le groupe phényle est éventuellement substitué avec jusqu'à trois groupes choisis parmi un atome d'halogène, un groupe alkyle en $C_{1-6}$, alkoxy en $C_{1-6}$, nitro, amino, mono- ou di-alkyle($C_{1-6}$amino et hydroxy; Y et Z, qui peuvent être identiques ou différents, représentent un atome d'oxygène ou de soufre; et X est un radical $—CH_2—$ ou un atome d'oxygène; et au moins un autre composé actif du point de vue anthelmintique choisi dans le groupe comprenant le fenbendazole, l'avermectine la piperazine, le morantel, le pyrantel, le levamisole et leurs sels acceptables du point de vue pharmaceutique, éventuellement avec un support acceptable du point de vue pharmaceutique.

2. Composition pharmaceutique suivant la revendication 1, caractérisée en ce que dans le composé de formule (I), R est un groupe cycloalkyle en $C_{3-8}$ ou cycloalcényle en $C_{5-8}$.

3. Composition pharmaceutique suivant les revendications 1 ou 2, caractérisée en ce que dans le composé de formule (I), Y et Z sont tous les deux un atomee d'oxygène.

4. Composition pharmaceutique suivant l'une quelconque des revendications 1 à 3, caractérisé en ce que l'autre composé anthelmintique actif est le pyrantel ou un sel de celui-ci acceptable du point de vue pharmaceutique.

5. Composition pharmaceutique suivant la revendication 4, caractérisée en ce que l'autre composé anthelmintique est le pamoate de pyrantel.

6. Composition pharmaceutique, caractérisé en ce qu'elle comprend la 2-(cyclohexylcarbonyl)-4-oxo-1,2,3,4,6,7,8,12b-octahydropyrazino[2,1-*a*][2]benzazépine et le pyrantel ou un sel de ceux-ci acceptable du point de vue pharmaceutique, avec un support acceptable du point de vue pharmaceutique.

7. Composition pharmaceutique suivant la revendication 6, caractérisée en ce que le pyrantel est sous la forme de pamoate.

8. Composition pharmaceutique suivant l'une quelconque des revendications 1 à 7, caractérisée en ce qu'elle est adaptée à l'administration orale.

9. Composition pharmaceutique suivant la revendication 8, caractérisée en ce qu'elle est sous la forme d'une grosse pilule, d'un comprimé ou d'une gélule.

10. Utilisation d'un composé de formule (I) suivant la revendication 1, et d'au moins un autre composé actif du point de vue anthelmintique choisi dans le groupe consistant en fenbendazole, avermectine, pipérazine, morantel, pyrantel, levamisole et leurs sels acceptables du point de vue pharmaceutique caractérisée en ce qu'ils servent à la fabrication d'un médicament pour le traitement de l'helminthiase chez l'homme et les animaux domestiques.